# EUROPEAN PATENT SPECIFICATION

(11) **EP 2 412 702 B1**
(45) Date of publication and mention of the grant of the patent: **03.12.2014**
(21) Application number: 10755975.9
(22) Date of filing: 12.03.2010
(51) Int. Cl.: C07C 253/30, C07C 255/31, C07C 255/41, C07B 61/00

(54) **METHOD FOR PRODUCING CYCLOPROPANECARBOXYLIC ACID ESTER**
VERFAHREN ZUR HERSTELLUNG VON CYCLOPROPANCARBONSÄUREESTER
PROCÉDÉ DE PRODUCTION D'UN ESTER D'ACIDE CYCLOPROPANECARBOXYLIQUE

(30) Priority: 23.03.2009 JP 2009069543
(43) Date of publication of application: 01.02.2012
(73) Proprietor: Sumitomo Chemical Company, Limited, Tokyo 104-8260 (JP)
(72) Inventor: OHSHITA, Jun, Nishinomiya-shi Hyogo 663-8245 (JP); UEKAWA, Toru, Toyonaka-shi Osaka 560-0021 (JP)
(74) Representative: Winter, Brandl, Fürniss, Hübner, Röss, Kaiser, Polte - Partnerschaft mbB
(86) International application number: PCT/JP2010/054721
(87) International publication number: WO 2010/110178

(56) References cited:
- JP-A- 55 033 496
- JP-A- 2004 002 363
- JP-A- 2008 239 597
- JP-A- 2009 185 019
- JP-A- 2009 185 019
- JP-B- 38 002 272
- KR-B1- 100 827 193
- US-A1- 2003 195 119
- US-A1- 2006 074 086
- AGRICULTURAL AND BIOLOGICAL CHEMISTRY vol. 34, no. 7, 1970, pages 1119 - 1125, XP055094130

## Description

### Technical Field

The present invention relates to a method for producing a cyclopropanecarboxylate.

### Background Art

US 2003/0,195,119 A1 discloses a method of reacting (4-methoxymethyl-2,3,5,6-tetrafluorobenzyl) 3-formyl-2,2-dimethylcyclopropanecarboxylate and diethyl (1-cyanoethyl)phosphonate, as a method for producing (4-methoxymethyl-2,3,5,6-tetrafluorobenzyl) 3-(2-cyano-1-propenyl)-2,2-dimethylcyclopropanecarboxylate which is a typical compound of cyclopropanecarboxylates represented by formula (III): 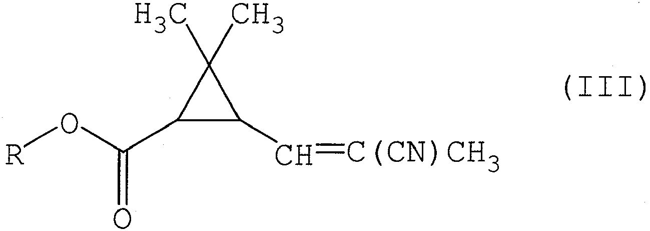 wherein, R represents a chain hydrocarbon group having 1 to 10 carbon atoms optionally having at least one member selected from the group consisting of halogen atoms, acyl groups having 2 to 7 carbon atoms optionally having a substituent, alkoxy groups having 1 to 7 carbon atoms optionally having a substituent, alkylthio groups having 1 to 3 carbon atoms and phenyl groups optionally having a substituent, a cyclic hydrocarbon group having 3 to 10 carbon atoms or a hydrogen atom.

### Disclosure of the Invention

The present invention provides
<1> A method for producing a cyclopropanecarboxylate represented by formula (III): 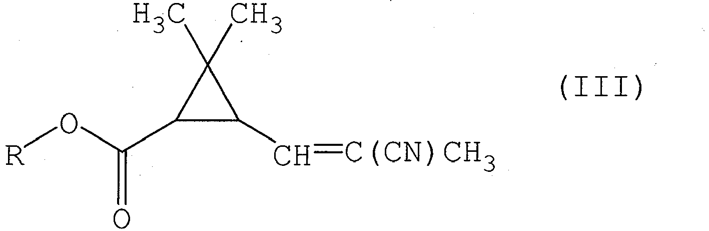 wherein, R represents a chain hydrocarbon group having 1 to 10 carbon atoms optionally having at least one member selected from the group consisting of halogen atoms, acyl groups having 2 to 7 carbon atoms optionally having a substituent, alkoxy groups having 1 to 7 carbon atoms optionally having a substituent, alkylthio groups having 1 to 3 carbon atoms and phenyl groups optionally having a substituent, a cyclic hydrocarbon group having 3 to 10 carbon atoms or a hydrogen atom;
   comprising reacting a cyclopropanecarboxylate represented by formula (I): 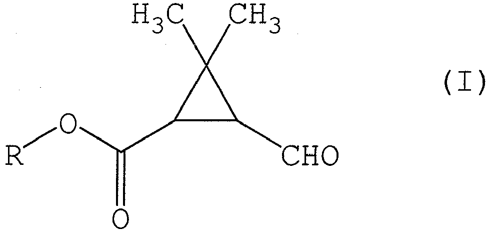 wherein, R represents the same meaning as described above; and 2-cyanopropionic acid in the presence of a base;
<2> The method according to <1> wherein the base is a primary amine or a secondary amine;
<3> The method according to <1> wherein the base is a secondary amine;
<4> The method according to <1>, <2> or <3> wherein R is a chain hydrocarbon group having 1 to 10 carbon atoms or a chain hydrocarbon group having 1 to 10 carbon atoms having a phenyl group optionally having a substituent;
<5> The method according to <1>, <2> or <3> wherein R is a methyl group or 2,3,5,6-tetrafluoro-4-methoxymethylbenzyl group;
and the like.

### Best Modes for Carrying Out the Invention

The present invention is a method for producing a cyclopropanecarboxylate represented by formula (III) (hereinafter, abbreviated as ester (III)): 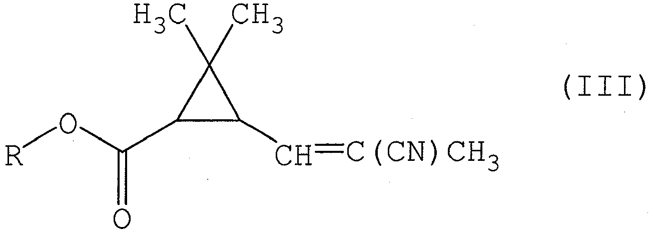 comprising reacting a cyclopropanecarboxylate represented by formula (I) (hereinafter, abbreviated as ester (I)): 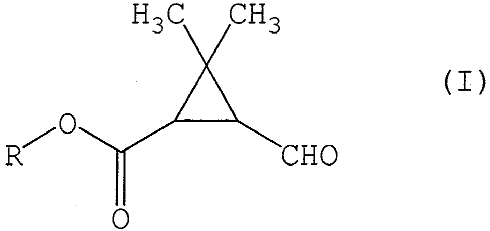 and 2-cyanopropionic acid and in the presence of a base.

In the above-described formula (I), R represents a chain hydrocarbon group having 1 to 10 carbon atoms optionally having at least one member selected from the group consisting of halogen atoms, acyl groups having 2 to 7 carbon atoms optionally having a substituent, alkoxy groups having 1 to 7 carbon atoms optionally having a substituent, alkylthio groups having 1 to 3 carbon atoms and phenyl groups optionally having a substituent, a cyclic hydrocarbon group having 3 to 10 carbon atoms or a hydrogen atom.

The halogen atom includes a chlorine atom and a fluorine atom, preferably a chlorine atom.

The acyl group having 2 to 7 carbon atoms optionally having a substituent includes unsubstituted acyl groups having 2 to 7 carbon atoms such as a phenacyl group and the like, and acyl groups having 2 to 7 carbon atoms having a halogen atom such as a p-bromophenacyl group and the like.

The alkoxy group having 1 to 7 carbon atoms optionally having a substituent includes unsubstituted alkoxy groups having 1 to 7 carbon atoms such as a methoxy group, ethoxy group, propoxy group and the like, alkoxy groups having 1 to 7 carbon atoms having an alkoxy group having 1 to 7 carbon atoms such as a methoxymethoxy group and the like, and alkoxy groups having 1 to 7 carbon atoms having a phenyl group such as a benzyloxy group and the like.

The alkylthio group having 1 to 3 carbon atoms includes a methylthio group.

The phenyl group optionally having a substituent includes phenyl groups which may have at least one member selected from the group consisting of halogen atoms, alkyl groups having 1 to 7 carbon atoms optionally having an alkoxy group having 1 to 7 carbon atoms, alkoxy groups having 1 to 7 carbon atoms optionally having an alkoxy group having 1 to 7 carbon atoms, a nitro group and a phthaloyl group; such as a 4-bromophenyl group, 4-methoxyphenyl group, 2,3-difluorophenyl group, 2,3,5-trifluorophenyl group, 2,3,5,6-tetrafluoro-4-methoxymethylphenyl group, 2,3,5,6-tetrafluoro-4-methylphenyl group, 2-nitrophenyl group, 4-nitrophenyl group, 2-(9,10-dioxo)anthryl group and the like.

The chain hydrocarbon group having 1 to 10 carbon atoms which may have at least one member selected from the group consisting of halogen atoms, acyl groups having 2 to 7 carbon atoms optionally having a substituent, alkoxy groups having 1 to 7 carbon atoms optionally having a substituent, alkylthio groups having 1 to 3 carbon atoms and phenyl groups optionally having a substituent includes
unsubstituted chain hydrocarbon groups having 1 to 10 carbon atoms such as a methyl group, ethyl group, propyl group, allyl group, propargyl group and the like;
chain hydrocarbon groups having 1 to 10 carbon atoms having at least on halogen atom such as a 2-chloroethyl group, 2,2,2-trichloroethyl group and the like;
chain hydrocarbon groups having 1 to 10 carbon atoms having an acyl groups having 2 to 7 carbon atoms optionally having a substituent such as a phenacyl group, p-bromophenacyl group and the like;
chain hydrocarbon groups having 1 to 10 carbon atoms having an alkoxy group having 1 to 7 carbon atoms optionally having a substituent such as a methoxymethyl group, methoxymethoxymethyl group, benzyloxymethyl group and the like;
chain hydrocarbon groups having 1 to 10 carbon atoms having an alkylthio group having 1 to 3 carbon atoms optionally having a substituent such as a methylthiomethyl group, 2-(methylthio)ethyl group and the like; and
chain hydrocarbon groups having 1 to 10 carbon atoms having a phenyl group optionally having a substituent such as a benzyl group, phenethyl group, 4-bromobenzyl group, 4-methoxybenzyl group, 2,3-difluorobenzyl group, 2,3,5-trifluorobenzyl group, a 2,3,5,6-tetrafluoro-4-methoxymethylbenzyl group, 2,3,5,6-tetrafluoro-4-methylbenzyl group, 2-nitrobenzyl group, 4-nitrobenzyl group, bis(o-nitrophenyl)methyl group, 2-(9,10-dioxo)anthranylmethyl group and the like.

The cyclic hydrocarbon group having 3 to 10 carbon atoms includes a cyclopropyl group, cyclopentyl group and cyclohexyl group.

Of them, R is preferably a chain hydrocarbon group having 1 to 10 carbon atoms or a chain hydrocarbon group having 1 to 10 carbon atoms having a phenyl group optionally having a substituent, more preferably a methyl group or 2,3,5,6-tetrafluoro-4-methoxymethylbenzyl group.

The ester (I) includes methyl 3-formyl-2,2-dimethylcyclopropanecarboxylate, ethyl 3-formyl-2,2-dimethylcyclopropanecarboxylate, propyl 3-formyl-2,2-dimethylcyclopropanecarboxylate, allyl 3-formyl-2,2-dimethylcyclopropanecarboxylate, propargyl 3-formyl-2,2-dimethylcyclopropanecarboxylate, (2-chloroethyl) 3-formyl-2,2-dimethylcyclopropanecarboxylate, (2,2,2-trichloroethyl) 3-formyl-2,2-dimethylcyclopropanecarboxylate, phenacylmethyl 3-formyl-2,2-dimethylcyclopropanecarboxylate, methoxymethyl 3-formyl-2,2-dimethylcyclopropanecarboxylate, methylthiomethyl 3-formyl-2,2-dimethylcyclopropanecarboxylate, benzyl 3-formyl-2,2-dimethylcyclopropanecarboxylate, (2,3-difluorobenzyl) 3-formyl-2,2-dimethylcyclopropanecarboxylate, (2,3,5-trifluorobenzyl) 3-formyl-2,2-dimethylcyclopropanecarboxylate, (2,3,5,6-tetrafluoro-4-methoxymethylbenzyl) 3-formyl-2,2-dimethylcyclopropanecarboxylate and (2,3,5,6-tetrafluoro-4-methylbenzyl) 3-formyl-2,2-dimethylcyclopropanecarboxylate.

The ester (I) can be obtained by known methods described in JP-A No. 2006-89427 and the like. The ester (I) may be a cis form in which a formyl group bonded to a cyclopropane ring and a group represented by -COOR bonded to a cyclopropane ring are present on the same side of the plane of the cyclopropane ring, or a trans form in which a formyl group bonded to a cyclopropane ring and a group represented by -COOR bonded to a cyclopropane ring are present on mutually opposite sides of the plane of the cyclopropane ring. The trans form is preferable, and a (1R, 3R) form is more preferable.

Commercially available 2-cyanopropionic acid can be used, and those produced by known methods described in Tetrahedron, 50, 4439 (1994) and the like can also be used.

The use amount of 2-cyanopropionic acid is usually 0.8 to 5 mol, preferably 1 to 2 mol with respect to 1 mol of the ester (I) .

The base includes alkali metal alkoxides such as sodium methoxide, potassium tert-butoxide and the like, alkali metal hydroxides such as sodium hydroxide, potassium hydroxide and the like, secondary amines such as pyrrolidine, piperidine, morpholine, dimethylamine, diethylamine and the like, and primary amines such as butylamine, hexylamine, aniline and the like. Of them, primary amines or secondary amines are preferable, secondary amines are more preferable, pyrrolidine and piperidine are particularly preferable.

The use amount of the base is usually 0.01 to 5 mol, preferably 0.1 to 0.5 mol with respect to 1 mol of the ester (I).

The reaction of the ester (I) and 2-cyanopropionic acid is usually carried out in the presence of a solvent. The solvent includes aromatic hydrocarbon solvents such as toluene, xylene, mesitylene, chlorobenzene and the like, aliphatic hydrocarbon solvents such as pentane, hexane, heptane, octane, cyclohexane and the like, ester solvents such as ethyl acetate, dimethyl carbonate and the like, halogenated hydrocarbon solvents such as dichloromethane, dichloroethane, carbon tetrachloride and the like, nitrile solvents such as acetonitrile, benzonitrile and the like, ether solvents such as diethyl ether, tetrahydrofuran, tert-butyl methyl ether and the like, alcohol solvents such as methanol, ethanol, isopropanol and the like, amide solvents such as N,N-dimethylformamide, N,N-dimethylacetamide and the like, pyridine, dimethyl sulfoxide, water and mixed solvents thereof. Of them, amide solvents are preferable, N,N-dimethylformamide is more preferable. Though the use amount of the solvent is not limited, it is usually 0.01 to 50 parts by weight, preferably 0.01 to 10 parts by weight with respect to 1 part by weight of the ester (I).

The reaction temperature is usually -20 to 120°C, preferably 50 to 120°C.

The reaction time is usually 5 minutes to 72 hours, though varying depending on the reaction temperature.

The progress of the reaction can be confirmed by usual means such as gas chromatography, high performance liquid chromatography and the like.

The reaction is carried out by mixing an ester (I), 2-cyanopropionic acid and a base, and the mixing order thereof is not restricted.

After completion of the reaction, a reaction mixture containing an ester (III) is obtained, and the ester (III) can be taken out, for example, by mixing the reaction mixture and water and if necessary a water-insoluble solvent, and concentrating the resultant organic layer. The taken out ester (III) may be further purified by usual purification means such as chromatography, distillation and the like.

Thus obtainable ester (III) includes methyl 3-(2-cyano-1-propenyl)-2,2-dimethylcyclopropanecarboxylate, ethyl 3-(2-cyano-1-propenyl)-2,2-dimethylcyclopropanecarboxylate, propyl 3-(2-cyano-1-propenyl)-2,2-dimethylcyclopropanecarboxylate, allyl 3-(2-cyano-1-propenyl)-2,2-dimethylcyclopropanecarboxylate, propargyl 3-(2-cyano-1-propenyl)-2,2-dimethylcyclopropanecarboxylate, (2-chloroethyl) 3-(2-cyano-1-propenyl)-2,2-dimethylcyclopropanecarboxylate, (2,2,2-trichloroethyl) 3-(2-cyano-1-propenyl)-2,2-dimethylcyclopropanecarboxylate, phenacylmethyl 3-(2-cyano-1-propenyl)-2,2-dimethylcyclopropanecarboxylate, methoxymethyl 3-(2-cyano-1-propenyl)-2,2-dimethylcyclopropanecarboxylate, methylthiomethyl 3-(2-cyano-1-propenyl)-2,2-dimethylcyclopropanecarboxylate, benzyl 3-(2-cyano-1-propenyl)-2,2-dimethylcyclopropanecarboxylate, (2,3-difluorobenzyl) 3-(2-cyano-1-propenyl)-2,2-dimethylcyclopropanecarboxylate, (2,3,5-trifluorobenzyl) 3-(2-cyano-1-propenyl)-2,2-dimethylcyclopropanecarboxylate, (2,3,5,6-tetrafluoro-4-methoxymethylbenzyl) 3-(2-cyano-1-propenyl)-2,2-dimethylcyclopropanecarboxylate and (2,3,5,6-tetrafluoro-4-methylbenzyl) 3-(2-cyano-1-propenyl)-2,2-dimethylcyclopropanecarboxylate.

In the above-described reaction, the steric configurations at 1-position and 3-position of a cyclopropane ring are kept. For example, in the case of use of a (1R,3R) form of the ester (I) in which the absolute configuration at 1-position of a cyclopropane ring to which a group represented by -COOR is bonded is R configuration and the absolute configuration at 3-position of a cyclopropane ring to which a formyl group is bonded is R configuration, a (1R,3R) form of the ester (III) is usually obtained in which the absolute configuration at 1-position of a cyclopropane ring to which a group represented by -COOR is bonded is R configuration and the absolute configuration at 3-position of a cyclopropane ring to which a 2-cyano-1-propenyl group is bonded is R configuration.

The ester (III) is usually a mixture of an ester (III) in which the steric configuration of a double bond of a 2-cyano-1-propenyl group at a 3-position of a cyclopropane ring is E configuration and an ester (III) in which its steric configuration is Z configuration.

### EXAMPLES

The present invention will be illustrated further in detail by examples below. Analysis was carried out by a high performance liquid chromatography internal standard method.

### Example 1

Under nitrogen atmosphere, a mixture of 20.0 g of methyl (1R,3R)-3-formyl-2,2-dimethylcyclopropanecarboxylate, 100 g of toluene and 5.56 g of piperidine was adjusted to 100°C. Into the mixture, 19.0 g of 2-cyanopropionic acid was dropped over a period of 45 minutes. The resultant mixture was stirred at the same temperature for 8 hours. To the resultant mixture was further added 1.11 g of piperidine, and the mixture was stirred at the same temperature for 1.5 hours. The resultant reaction mixture was cooled down to room temperature, then, 100 g of a 10 wt% sulfuric acid aqueous solution was added, and the mixture was separated into an organic layer and an aqueous layer. The resultant aqueous layer was extracted with 100 g of toluene, and the resultant toluene layer was mixed with the organic layer obtained above. The resultant solution was washed with 100 g of a 3.0 wt% sodium hydrogen carbonate aqueous solution, further washed with 100 g of water. The resultant solution was concentrated under reduced condition to obtain 24.5 g of a coarse product containing methyl (1R,3R)-3-(2-cyano-1-propenyl)-2,2-dimethylcyclopropanecarb oxylate. The coarse product was analyzed by a gas chromatography internal standard method to find that the content of methyl (1R,3R)-3-(2-cyano-1-propenyl)-2,2-dimethylcyclopropanecarb oxylate was 72% and the ratio of E form to Z form (hereinafter, abbreviated as E/Z ratio) was 2/1. The yield (total of E form and Z form) of methyl (1R,3R)-3-(2-cyano-1-propenyl)-2,2-dimethylcyclopropanecarb oxylate was 72%.

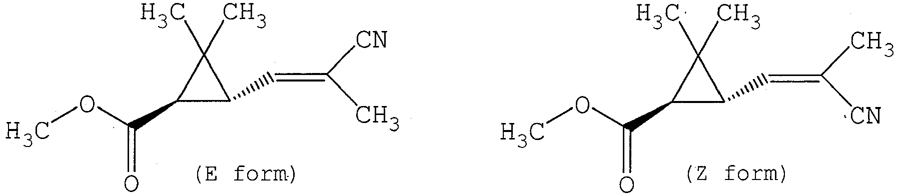

### Example 2

Under nitrogen atmosphere, a mixture of 0.50 g of methyl (1R,3R)-3-formyl-2,2-dimethylcyclopropanecarboxylate, 2.5 g of toluene, 0.50 g of N,N-dimethylformamide, 0.50 g of 2-cyanopropionic acid and 0.14 g of piperidine was stirred at an internal temperature of 100°C for 5.5 hours to obtain a reaction mixture containing methyl (1R,3R)-3-(2-cyano-1-propenyl)-2,2-dimethylcyclopropanecarb oxylate. The reaction mixture was analyzed by a gas chromatography internal standard method to find that the E/Z ratio of methyl (1R,3R)-3-(2-cyano-1-propenyl)-2,2-dimethylcyclopropanecarb oxylate was 1.4/1 and the yield (total of E form and Z form) of methyl (1R,3R)-3-(2-cyano-1-propenyl)-2,2-dimethylcyclopropanecarb oxylate was 89%.

### Examples 3 to 5

The reactions were carried out in the same manner as in Example 2 excepting that solvents described in Table 1 were used instead of 2.5 g of toluene and 0.50 g of N,N-dimethylformamide, bases described in Table 1 were used instead of 0.14 g of piperidine, and the mixtures were stirred at internal temperatures for times each described in Table 1, in Example 2, to obtain reaction mixtures containing methyl (1R,3R)-3-(2-cyano-1-propenyl)-2,2-dimethylcyclopropanecarb oxylate. The results are shown in Table 1. In Table 1, "DMF" represents N,N-dimethylformamide.

**[Table 1]**

| | Example 3 | Example 4 | Example 5 |
|---|---|---|---|
| Solvent | toluene 1.1 g | DMF 1.9 g | toluene 2.5 g |
| | DMF 1.9 g | | |
| Base | pyrrolidine 0.11 g | pyrrolidine 0.11 g | hexylamine 0.16 g |
| Internal temperature (°C) | 80 | 80 | 80 |
| Time (Hr) | 3.5 | 1.5 | 6.5 |
| E/Z ratio | 1.7/1 | 1.8/1 | 1/1.9 |
| Yield (%) | 89 | 90 | 75 |

### Example 6

Under nitrogen atmosphere, a mixture of 1.0 g of (2,3,5,6-tetrafluoro-4-methoxymethylbenzyl) (1R,3R)-3-formyl-2,2-dimethylcyclopropanecarboxylate, 5.0 g of toluene, 1.0 g of N,N-dimethylformamide, 0.41 g of 2-cyanopropionic acid and 92 mg of pyrrolidine was stirred at an internal temperature of 80°C for 6.5 hours. The resultant reaction mixture was cooled down to room temperature, then, 5.0 g of a 10 wt% sulfuric acid aqueous solution was added, and the mixture was separated into an organic layer and an aqueous layer. The resultant aqueous layer was extracted with 5.0 g of toluene, and the resultant toluene layer was mixed with the organic layer obtained above. The resultant solution was washed with 5.0 g of a 3.0 wt% sodium hydrogen carbonate aqueous solution, further washed with 5.0 g of water. The resultant solution was concentrated under reduced condition to obtain a coarse product containing (2,3,5,6-tetrafluoro-4-methoxymethylbenzyl) (1R,3R)-3-(2-cyano-1-propenyl)-2,2-dimethylcyclopropanecarb oxylate . ¹H-NMR of the coarse product was measured to find that the E/Z ratio was 1.9/1. The coarse product was purified by silica gel column chromatography to obtain 0.57 g of an E form of (2,3,5,6-tetrafluoro-4-methoxymethylbenzyl) (1R,3R)-3-(2-cyano-1-propenyl)-2,2-dimethylcyclopropanecarb oxylate and 0.30 g of a Z form of (2,3,5,6-tetrafluoro-4-methoxymethylbenzyl) (1R,3R)-3-(2-cyano-1-propenyl)-2,2-dimethylcyclopropanecarb oxylate. The yield (total of Z form and E form) was 79% (E/Z = 1.9/1).

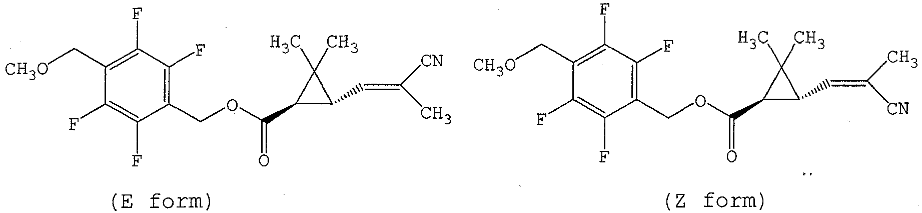

### Industrial Applicability

The present invention is capable of producing a cyclopropanecarboxylate represented by formula (III) in good yield which is a compound useful as a pest control agent and an intermediate thereof, thus, the present invention is industrially advantageous.

## Claims

1. A method for producing a cyclopropanecarboxylate represented by formula (III): 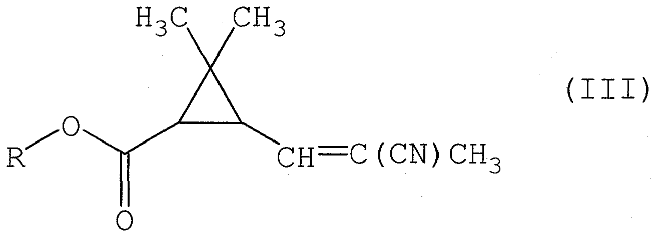 wherein, R represents a chain hydrocarbon group having 1 to 10 carbon atoms optionally having at least one member selected from the group consisting of halogen atoms, acyl groups having 2 to 7 carbon atoms optionally having a substituent, alkoxy groups having 1 to 7 carbon atoms optionally having a substituent, alkylthio groups having 1 to 3 carbon atoms and phenyl groups optionally having a substituent, a cyclic hydrocarbon group having 3 to 10 carbon atoms or a hydrogen atom;
comprising reacting a cyclopropanecarboxylate represented by formula (I): 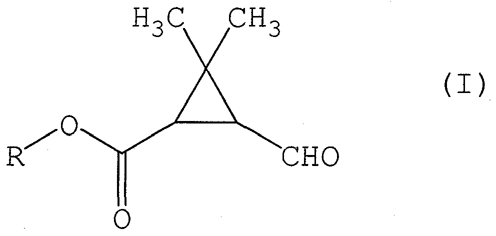 wherein, R represents the same meaning as described above; and 2-cyanopropionic acid in the presence of a base.

2. The method according to Claim 1, wherein the base is a primary amine or a secondary amine.

3. The method according to Claim 1, wherein the base is a secondary amine.

4. The method according to Claim 1, wherein R is a chain hydrocarbon group having 1 to 10 carbon atoms or a chain hydrocarbon group having 1 to 10 carbon atoms having a phenyl group optionally having a substituent.

5. The method according to Claim 1, wherein R is a methyl group or 2,3,5,6-tetrafluoro-4-methoxymethylbenzyl group.

## Patentansprüche

1. Verfahren zur Herstellung eines Cyclopropancarboxylats der Formel (III): 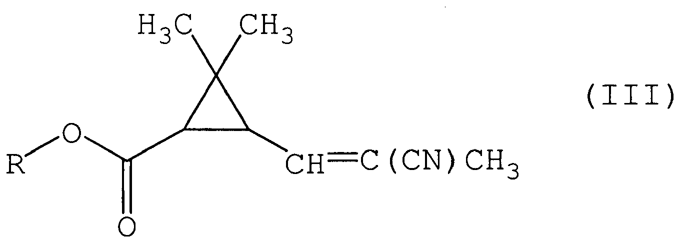 worin R eine Kohlenwasserstoffkette mit 1 bis 10 Kohlenstoffatomen, die gegebenenfalls wenigstens ein Element aufweist, das ausgewählt ist aus der Gruppe bestehend aus Halogenatomen, gegebenenfalls substituierten Acylgruppen mit 2 bis 7 Kohlenstoffatomen, gegebenenfalls substituierten Alkoxygruppen 1 bis 7 Kohlenstoffatomen, Alkylthiogruppen mit 1 bis 3 Kohlenstoffatomen und gegebenenfalls substituierten Phenylgruppen, eine cyclische Kohlenwasserstoffgruppe mit 3 bis 10 Kohlenstoffatomen oder ein Wasserstoffatom bedeutet;
umfassend die Umsetzung eines Cyclopropancarboxylats der Formel (I): 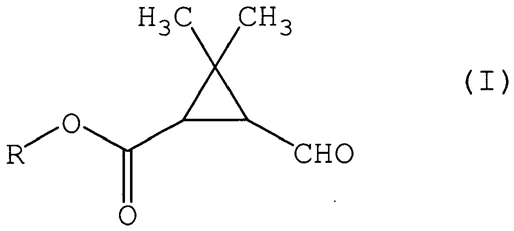 worin R die obige Bedeutung hat;
mit 2-Cyanpropionsäure in Gegenwart einer Base.

2. Verfahren nach Anspruch 1, wobei die Base ein primäres Amin oder ein sekundäres Amin ist.

3. Verfahren nach Anspruch 1, wobei die Base ein primäres Amin oder ein sekundäres Amin ist.

4. Verfahren nach Anspruch 1, wobei R eine Kohlenwasserstoffkette mit 1 bis 10 Kohlenstoffatomen oder eine Kohlenwasserstoffkette mit 1 bis 10 Kohlenstoffatomen mit einer gegebenenfalls substituierten Phenylgruppe ist.

5. Verfahren nach Anspruch 1, wobei R eine Methylgruppe oder eine 2,3,5,6-Tetrafluor-4-methoxymethylbenzylgruppe ist.

## Revendications

1. Procédé pour produire un cyclopropanecarboxylate représenté par la formule (III) : 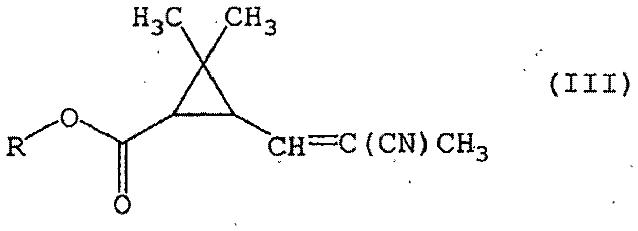 où, R représente un groupe hydrocarboné à chaîne ayant 1 à 10 atomes de carbone ayant éventuellement au moins un membre choisi dans le groupe consistant en les atomes d'halogène, les groupes acyle ayant 2 à 7 atomes de carbone ayant éventuellement un substituant, les groupes alcoxy ayant 1 à 7 atomes de carbone ayant éventuellement un substituant, les groupes alkylthio ayant 1 à 3 atomes de carbone et les groupes phényle ayant éventuellement un substituant, un groupe hydrocarboné cyclique ayant 3 à 10 atomes de carbone ou un atome d'hydrogène ;
comprenant la réaction d'un cyclopropanecarboxylate représenté par la formule (I) : 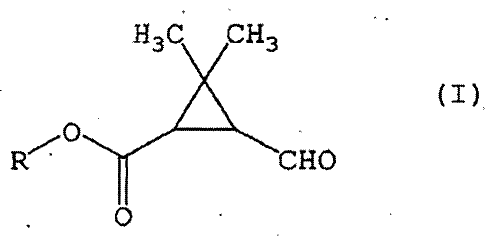 où R représente la même signification que décrit ci-dessus ; et de l'acide 2-cyanopropionique en présence d'une base.

2. Procédé selon la revendication 1, où la base est une amine primaire ou une amine secondaire.

3. Procédé selon la revendication 1, où la base est une amine secondaire.

4. Procédé selon la revendication 1, où R est un groupe hydrocarboné à chaîne ayant 1 à 10 atomes de carbone ou un groupe hydrocarboné à chaîne ayant 1 à 10 atomes de carbone ayant un groupe phényle ayant éventuellement un substituant.

5. Procédé selon la revendication 1, où R est un groupe méthyle ou un groupe 2,3,5,6-tétrafluoro-4-méthoxyméthylbenzyle.
